# FASCICULE DE BREVET EUROPEEN

(11) **EP 4 178 969 B1**
(45) Date de publication et mention de la délivrance du brevet: **07.02.2024**
(21) Numéro de dépôt: 21740069.6
(22) Date de dépôt: 08.07.2021
(51) Int. Cl.: C07H 1/00, C07H 17/07

(54) **PROCÉDÉ DE PRÉPARATION DE LA DIOSMINE**
VERFAHREN ZUR HERSTELLUNG VON DIOSMIN
METHOD FOR PREPARING DIOSMIN

(30) Priorité: 09.07.2020 EP 20315345
(43) Date de publication de la demande: 17.05.2023
(73) Titulaire: Les Laboratoires Servier, 92284 Suresnes Cedex (FR)
(72) Inventeur: SCHIAVI, Bruno, 76210 Gruchet le Valasse (FR); BESCOND, Philippe, 91770 Saint-Vrain (FR); MOUCHET, Patrick, 76760 ECTOT-L'AUBER (FR)
(86) Numéro de dépôt international: PCT/EP2021/068970
(87) Numéro de publication internationale: WO 2022/008647

(56) Documents cités:
- WO-A1-2016/124585
- FR-A1- 2 311 028
- FR-A1- 2 782 518

## Description

La présente invention concerne un procédé de préparation de la diosmine. La diosmine est le composé de formule (I) :

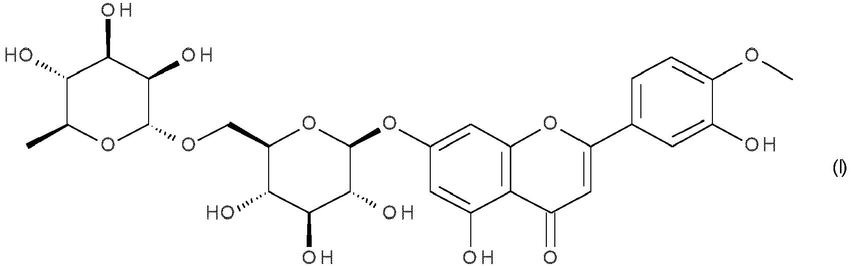

La diosmine est utilisée dans le traitement des maladies veineuses, comme l'insuffisance veineuse chronique ou les maladies hémorroïdales.

Elle est également le composant majoritaire de la fraction flavonoïque purifiée micronisée, ou FFPM (Daflon^{®}).

La diosmine est synthétisée par oxydation de l'hespéridine. L'hespéridine est le composé de formule (II) :

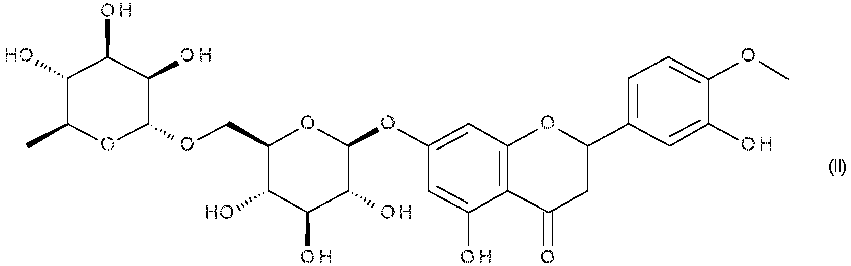

L'hespéridine est obtenue à partir de substances naturelles (orangettes). La diversité des orangettes utilisées conduit à des hespéridines de pureté inégale, contenant d'autres flavonoïdes avec des teneurs variables. En particulier, l'hespéridine peut contenir jusqu'à 4% d'isonaringine, qui se transforme en isorhoifoline par oxydation.

La diosmine contient donc en général d'autres flavonoïdes, dont certains proviennent de l'oxydation des flavonoïdes présents dans l'hespéridine de départ, et d'autres sont des produits secondaires de réaction.

Compte tenu de l'intérêt pharmaceutique de la diosmine, il est primordial de l'obtenir avec un excellent rendement et la pureté requise, quelle que soit la source d'hespéridine.

Les spécifications imposées par la Pharmacopée Européenne sont les suivantes :

| Substances | Spécifications diosmine (Pharmacopée Européenne) |
|---|---|
| Diosmine | 90,0 à 102,0 % |
| Hespéridine | < 4,0% |
| Diosmétine | < 2,0 % |
| Isorhoifoline | < 3,0 % |
| Linarine | < 3,0 % |
| 6-Iododiosmine | < 0,6 % |

En particulier, il est primordial que la diosmine obtenue contienne moins de 0,6% de 6-iododiosmine et moins de 3,0% d'isorhoifoline.

Des procédés de préparation de la diosmine à partir d'hespéridine ont été décrits dans la littérature.

FR2311028 décrit un procédé d'obtention de la diosmine par acétylation de l'hespéridine puis oxydation de l'hespéridine acétylée par bromation, hydrolyse basique et isolement. La diosmine brute ainsi obtenue est purifiée par une étape de retraitement utilisant de la pyridine.

Ce procédé n'est pas idéal, car le rendement n'est que de 65%. De plus, il utilise de la pyridine, solvant cancérogène de classe 3, tout comme FR2782518 qui décrit un procédé de fabrication de la diosmine à partir de l'hespéridine par réaction avec de l'iode et de la pyridine.

La demande de brevet WO2016/124585 présente l'avantage de ne pas utiliser de solvants organiques tels que la pyridine. Cependant, le procédé qui y est décrit ne permet pas d'obtenir la diosmine avec la pureté requise, lorsque l'hespéridine utilisée contient une quantité importante d'isonaringine.

L'un des problèmes de la présente invention était de minimiser la teneur en 6-iododiosmine dans la diosmine obtenue, tout en s'affranchissant de l'utilisation de solvants de classe 3 tels que la pyridine.

Un autre problème de la présente invention était de minimiser la teneur en isorhoifoline dans la diosmine obtenue, tout en s'affranchissant de l'utilisation de solvants de classe 3 tels que la pyridine, et ce, à partir d'une hespéridine contenant jusqu'à 4% d'isonaringine.

Plus spécifiquement, la présente invention concerne un procédé de préparation de la diosmine par
a) acétylation de l'hespéridine,
b) oxydation de l'hespéridine acétylée en diosmine acétylée par un donneur d'iode, à une température de 90 à 120°C,
c) chauffage de la diosmine acétylée, en autoclave à une pression de 5 à 8 bars, au reflux d'un alcool tel que le méthanol, l'éthanol ou l'isopropanol, en présence d'une base choisie parmi l'acétate de sodium ou de potassium, l'hydroxyde de sodium, de potassium ou de lithium, le carbonate de potassium, le méthanolate de sodium ou l'éthanolate de sodium, seule ou en mélange avec une autre de ces bases, puis
d) déprotection de la diosmine acétylée en diosmine par chauffage en présence d'une base choisie parmi l'hydroxyde de sodium, de potassium ou de lithium, le carbonate de potassium, le méthanolate de sodium ou l'éthanolate de sodium, seule ou en mélange avec de l'acétate de sodium ou de potassium,
e) purification par traitement base/acide.

Selon un mode de réalisation de la présente invention, la diosmine obtenue contient d'autres flavonoïdes tels que l'hespéridine, l'isorhoifoline, la linarine ou la diosmétine.

Selon un mode de réalisation de la présente invention, l'étape d'acétylation (a) est effectuée par réaction de l'hespéridine avec de l'anhydride acétique et de l'acétate de potassium ou de sodium.

La réaction d'acétylation est préférentiellement réalisée à une température entre 40°C et 135°C.

La quantité d'anhydride acétique est préférentiellement entre 8 et 10 équivalents molaires par rapport à l'hespéridine engagée.

Le donneur d'iode utilisé dans l'étape d'oxydation (b) est préférentiellement choisi parmi NaI/H₂O₂, KI/H₂O₂, TBAI/H₂O₂ et NaI/I₂ (préf 9/1) /H₂O₂.

La quantité de NaI est préférentiellement de 0,05 à 0,20 équivalent molaire par rapport à l'hespéridine engagée.

La quantité d'eau oxygénée est préférentiellement de 1,0 à 1,2 équivalent molaire par rapport à l'hespéridine engagée.

Selon un mode de réalisation de la présente invention, la diosmine acétylée obtenue à l'issue de l'étape d'oxydation (b) est isolée, préférentiellement par précipitation dans l'eau avant d'être engagée dans l'étape c).

Selon un mode de réalisation de la présente invention, la base utilisée pour l'étape c) est une solution aqueuse d'hydroxyde de sodium ou de potassium, une solution aqueuse d'acétate de sodium ou de potassium, ou un mélange d'hydroxyde de sodium ou de potassium et d'acétate de sodium ou de potassium en solution aqueuse.

L'acétate de sodium ou de potassium utilisé pour l'étape c) peut être généré *in situ* par neutralisation de l'acide acétique résiduel présent dans la diosmine acétylée par l'hydroxyde de sodium ou de potassium.

La quantité de base utilisée dans l'étape (c) est préférentiellement entre 0,5 et 2,5 équivalent molaire par rapport à l'hespéridine engagée.

Selon un mode de réalisation de la présente invention, la base ajoutée dans l'étape de désacétylation (d) est l'hydroxyde de sodium ou de potassium.

La quantité de base ajoutée dans l'étape de désacétylation (d) est préférentiellement entre 2 et 4,5 équivalents molaires par rapport à l'hespéridine engagée.

Selon un mode de réalisation de la présente invention, le traitement base/acide (étape e) est réalisé par passage en solution dans l'eau en présence d'une base telle que l'hydroxyde de sodium, puis précipitation par salification avec un acide, tel que l'acide sulfurique.

Les exemples suivants illustrent l'invention.

### Abréviations :

- éq mol: équivalents molaires (par rapport à l'hespéridine)
- HPLC: High Performance Liquid Chromatography (Chromatographie en phase liquide à haute performance)
- m/m: rapport exprimé en masse/masse
- TBAI: iodure de tétra n-buylammonium
- vol: équivalents volumiques (par rapport à l'hespéridine)

### EXEMPLE 1 : Diosmine

### Stade A : Diosmine acétylée

Charger dans un réacteur entre 20-25°C l'acétate de potassium (98,6 mmol) et l'anhydride acétique (2996,2 mmol).

Chauffer la suspension sous agitation à 40°C puis charger de l'hespéridine (2 x 163,8 mmol; titre HPLC : 91,3%, isonaringine 3,8%). Poursuivre l'agitation en chauffant à 40°C, puis chauffer à 132°C en 45 min sous agitation. Le mélange passe en solution limpide en fin de chauffage. Laisser agiter la solution pendant 60 mn à 132°C puis refroidir à 105°C.

Charger la solution aqueuse d'iodure de sodium (33 mmol dans 20g d'eau). Couler à 105°C l'eau oxygénée à 35% (341,5 mmol) stabilisée à 0,1% d'acide sulfurique.

Laisser agiter pendant 30 min à 105°C puis refroidir à 100°C sous agitation et précipiter dans un becher contenant de l'eau (environ 7 vol), sous agitation mécanique à 20-40°C.

Après 30 mn d'agitation entre 20-40°C, filtrer sous vide, laver le gâteau par de l'eau (9 vol; puis 2x 2 vol). Expurger pendant 16h sous vide entre 20-25°C.

### Stade B : Diosmine

Dans un autoclave, charger la diosmine acétylée obtenue au stade A et du méthanol (3,5 vol). Mettre en agitation puis chauffer au reflux à une pression de 5 bars. Après 15 mn à reflux, introduire de la soude en solution aqueuse à 30% (1,2 éq mol). Chauffer au reflux pendant 30 mn puis refroidir à 50°C à pression normale et introduire de la soude en solution aqueuse à 30% (2,4 éq mol). Après 2h à 50°C, refroidir à 20°C puis filtrer et laver le gâteau avec du méthanol (2 x 3 vol).

Dissoudre la diosmine brute dans 2,5 éq mol d'hydroxyde de sodium et d'eau (2,5 vol) à 20°C Ajouter de l'acide sulfurique pour ajuster le pH entre 2 et 4. Maintenir 30 mn à 20°C, filtrer, laver 2 fois par de l'eau (2 x 5vol) et sécher.
Rendement à partir de l'hespéridine : 83,8%
Pureté (HPLC): 90,6%
Teneur en 6-iododiosmine : 0,3%
Teneur en isorhoifoline : 2,0%.

### EXEMPLE 2 : Diosmine

### Stade A : Diosmine acétylée

Charger dans un réacteur entre 20-25°C l'acétate de potassium (207,1 mmol) et l'anhydride acétique (6291,9 mmol).

Chauffer la suspension sous agitation à 100°C puis charger l'hespéridine (5 x 137,6 mmol ; titre HPLC : 91,7% et isonaringine 3,6%). Poursuivre l'agitation en chauffant à 100°C, puis chauffer à 132°C en 15 min sous agitation. Le mélange passe en solution limpide en fin de chauffage. Laisser agiter la solution pendant 120 mn à 132°C puis refroidir à 105°C.

Charger la solution aqueuse d'iodure de sodium (68,8 mmol dans 40 g d'eau). Couler à 105°C l'eau oxygénée à 35% (717,1 mmol) stabilisée à 0,1% d'acide sulfurique.

Laisser agiter pendant 30 min à 105°C puis refroidir à 100°C sous agitation et précipiter dans un becher contenant de l'eau (environ 7 vol), sous agitation mécanique à 20-40°C.

Après 30 mn d'agitation entre 20-40°C, filtrer sous vide, laver le gâteau par de l'eau (9 vol ; puis 2x 2 vol). Expurger pendant 16h sous vide entre 20-25°C.

### Stade B : Diosmine

Dans un autoclave, charger la diosmine acétylée obtenue au stade A et du méthanol (3,5 vol). Mettre en agitation puis chauffer au reflux à une pression de 5 bars. Après 15 mn à reflux, introduire de la soude en solution aqueuse à 30% (1,55 éq mol.). Chauffer au reflux pendant 30 mn puis refroidir à 50°C à pression normale et introduire de la soude en solution aqueuse à 30% (2,4 éq mol). Après 2h à 50°C, refroidir à 20°C puis filtrer et laver le gâteau avec du méthanol (2 x 3 vol).

Dissoudre la diosmine brute dans 2,5 éq mol d'hydroxyde de sodium et de l'eau (2,5 vol) à 20°C.

Ajouter de l'acide sulfurique pour ajuster le pH entre 2 et 4. Maintenir 30 mn à 20°C, filtrer, laver 2 fois par de l'eau (2 x 5 vol) et sécher.
Rendement à partir de l'hespéridine : 81,2%
Pureté (HPLC): 90,4%
Teneur en 6-iododiosmine : 0,29%
Teneur en isorhoifoline : 2,2%.

### EXEMPLE 3 : Diosmine

Dans un autoclave, charger la diosmine acétylée obtenue au stade A de l'Exemple 1 et du méthanol (3,5 vol), ajouter 2 éq mol d'une solution aqueuse d'acétate de potassium puis chauffer au reflux à une pression de 7 à 8 bars. Refroidir ensuite à 50°C et introduire une solution aqueuse de potasse (4,2 éq mol). Après un contact à 50°C, refroidir à 20°C, puis filtrer et laver avec du méthanol (2 x 1,5 vol).

Dissoudre la diosmine brute dans 2,5 éq mol d'hydroxyde de sodium et d'eau (2,5 vol) à 20°C. ajouter de l'acide sulfurique pour ajuster le pH entre 2 et 4. Maintenir 30 min à 20°C puis filtrer, laver 2 fois par de l'eau (2 x 5 vol) et sécher.
Rendement à partir de l'hespéridine : 87,7%
Pureté (HPLC) : 90,1%
Teneur en 6-iododiosmine : non détectée (< 0,10%)

### EXEMPLE 4 : Diosmine acétylée avec différents donneurs d'iode

Dans un tricol de 25 mL équipé d'une agitation ovoïde et d'un pousse-seringue, charger 10g d'hespéridine, 0,5 g d'acétate de potassium et 14mL/15,6g d'anhydride acétique. Amener progressivement la température à 132°C et laisser 1h à 130°C.

Refroidir à environ 90°C puis charger 0,322 g d'iodure de sodium ou le donneur d'iode équivalent et 2,258 g d'eau. Chauffer à 105°C puis additionner l'eau oxygénée à 35% (1,1835 mL/1,645g) et 5,161 g d'eau.

| Essai | 3a | 3b | 3c | 3d |
|---|---|---|---|---|
| Hespéridine | 10 g | 2g | 2g | 2g |
| Anhydride acétique | 9,146 éq mol | 9,146 éq mol | 9,146 éq mol | 9,146 éq mol |
| Acétate de potassium | 0,327 éq mol | 0,327 éq mol | 0,327 éq mol | 0,327 éq mol |
| HzOz 35% | 1,036 éq mol | 1,033 éq mol | 1,033 éq mol | 1,033 éq mol |
| H₂SO₄ | / | 0,00109 éq mol | 0,00109 éq mol | 0,00109 éq mol |
| Donneur d'iode | NaI | KI | TBAI | NaI / I₂ 9/1 |
| | 0,133 éq mol | 0,133 éq mol | 0,133 éq mol | 0,120/0,013 éq mol |
| Diosmine acétylée | 94% | > 97% | > 98% | > 97% |

### EXEMPLE 5 : Diosmine avec différentes bases

Dans un autoclave, charger la diosmine acétylée obtenue au Stade A de l'Exemple 2 et du méthanol (3,5 vol). Mettre en agitation puis chauffer au reflux à une pression de 7 bars. Après 15 mn à reflux, introduire la base en solution aqueuse à 30% (1,2 éq mol). Chauffer au reflux pendant 30 mn puis refroidir à 50°C à pression normale et introduire la base en solution aqueuse à 30% (2,4 éq mol). Après 2h à 50°C, refroidir à 20°C puis filtrer et laver le gâteau avec du méthanol (2 x 3 vol).

| Essai | 4a | 4b | 4c | 4d | 4e |
|---|---|---|---|---|---|
| Base | CH₃ONa | NaOH | LiOH | KOH | K₂CO₃ |
| Rendement / Hespéridine engagée | 84% | 84% | 85% | 84% | 80% |
| Diosmine | 89,8 % | 90,9 % | 90,4 % | 90,8 % | 92,4 % |
| Isorhoifoline | 2,6 % | 2,0 % | 2,1 % | 2,0 % | 2,1 % |
| 6-Iododiosmine | 0,45 % | 0,46 % | 0,42 % | 0,62 % | 0,54 % |

### EXEMPLE 6 (comparatif) : Reproduction du procédé de WO 2016/124585

40 g d'anhydride acétique, 0,75 g d'acétate de potassium et 30 g d'hespéridine (pureté 91,3% ; isonaringine 3,8%) sont chargés dans un réacteur. Le milieu réactionnel est ensuite chauffé à 115-120°C, en maintenant cette température pendant une heure environ, puis le milieu est refroidi à 60-70° C.

Une solution d'iodure de sodium (0,9 g) dans l'eau (6 mL) est ajoutée, et le milieu réactionnel est chauffé à reflux. Puis on ajoute au mélange réactionnel une solution de 35 mL d'eau oxygénée à 5,4 % (massique) stabilisée par de l'acide sulfurique, en maintenant le reflux. Ensuite, le milieu réactionnel est refroidi à 40-50°C et de l'hydroxyde de potassium (10 g) est ajouté au mélange réactionnel ; le pH est alors à 4. Le mélange est ensuite chauffé à 115-120°C pendant 3 heures, puis refroidi à 30° C.

Le mélange réactionnel est ajouté à un réacteur contenant une solution aqueuse de soude 2N (300 mL). Après 1h30, de l'acide sulfurique est ajouté jusqu'à ce que le pH atteigne 7,5. Le précipité est ensuite filtré et lavé à l'eau, pour obtenir de la diosmine brute humide.

La diosmine brute ainsi obtenue est cristallisée en la dissolvant dans une solution aqueuse d'hydroxyde de sodium, puis en l'acidifiant avec de l'acide sulfurique jusqu'à la précipitation du produit.

Le solide est filtré, lavé à l'eau et séché.

Analyse (HPLC) :

| Substances | Pourcentage dans le produit de l'Exemple 5 | Spécifications diosmine (Pharmacopée Européenne) |
|---|---|---|
| Diosmine | 87,1 % | 90,0 à 102,0 % |
| Isorhoifoline | 3,6 % | < 3,0 % |
| 6-Iododiosmine | 0,99 % | < 0,6 % |

## Revendications

1. Procédé de préparation de la diosmine par
a) acétylation de l'hespéridine,
b) oxydation de l'hespéridine acétylée en diosmine acétylée par un donneur d'iode, à une température de 90 à 120°C,
c) chauffage de la diosmine acétylée, en autoclave à une pression de 5 à 8 bars, au reflux d'un alcool, en présence d'une base choisie parmi l'acétate de sodium ou de potassium, l'hydroxyde de sodium, de potassium ou de lithium, le carbonate de potassium, le méthanolate de sodium ou l'éthanolate de sodium, seule ou en mélange avec une autre de ces bases, puis
d) déprotection de la diosmine acétylée en diosmine par chauffage en présence d'une base choisie parmi l'hydroxyde de sodium, de potassium ou de lithium, le carbonate de potassium, le méthanolate de sodium ou l'éthanolate de sodium, seule ou en mélange avec de l'acétate de sodium ou de potassium,
e) purification par traitement base/acide.

2. Procédé selon la revendication 1, dans lequel la diosmine obtenue contient d'autres flavonoïdes.

3. Procédé selon l'une quelconque des revendications 1 ou 2, dans lequel la diosmine obtenue contient moins de 0,6% de 6-iododiosmine et moins de 3,0% d'isorhoifoline.

4. Procédé selon l'une quelconque des revendications 1 à 3, dans lequel la quantité d'anhydride acétique est entre 8 et 10 équivalents molaires par rapport à l'hespéridine engagée.

5. Procédé selon l'une quelconque des revendications 1 à 4, dans lequel la réaction d'acétylation (a) est réalisée à une température entre 40°C et 135°C.

6. Procédé selon l'une quelconque des revendications 1 à 5, dans lequel le donneur d'iode est choisi parmi NaI/H₂O₂, KI/H₂O₂, TBAI/H₂O₂ et NaI/I₂ /H₂O₂.

7. Procédé selon la revendication 6, dans lequel le donneur d'iode est NaI en quantité de 0,05 à 0,2 équivalent molaire par rapport à l'hespéridine engagée.

8. Procédé selon l'une quelconque des revendications 1 à 7, dans lequel la quantité d'eau oxygénée est de 1,0 à 1,2 équivalent molaire par rapport à l'hespéridine engagée.

9. Procédé selon l'une quelconque des revendications 1 à 8, dans lequel la diosmine acétylée obtenue à l'issue de l'étape d'oxydation (b) est isolée par précipitation dans l'eau avant d'être engagée dans l'étape c).

10. Procédé selon l'une quelconque des revendications 1 à 9, dans lequel la base utilisée pour l'étape c) est l'hydroxyde de sodium ou de potassium en solution aqueuse, l'acétate de sodium ou de potassium en solution aqueuse, ou un mélange d'hydroxyde de sodium ou de potassium et d'acétate de sodium ou de potassium en solution aqueuse.

11. Procédé selon l'une quelconque des revendications 1 à 10, dans lequel l'alcool utilisé pour l'étape c) est le méthanol, l'éthanol ou l'isopropanol.

12. Procédé selon l'une quelconque des revendications 1 à 11, dans lequel la quantité de base utilisée dans l'étape (c) est entre 0,5 et 2,5 équivalent molaire par rapport à l'hespéridine engagée.

13. Procédé selon l'une quelconque des revendications 1 à 12, dans lequel la quantité de base ajoutée dans l'étape de désacétylation (d) est entre 2 et 4,5 équivalents molaires par rapport à l'hespéridine engagée.

## Patentansprüche

1. Verfahren zur Herstellung von Diosmin durch
a) Acetylierung von Hesperidin,
b) Oxidation des acetylierten Hesperidins zu acetyliertem Diosmin durch einen Joddonator bei einer Temperatur von 90 bis 120 °C,
c) Erhitzen des acetylierten Diosmins in einem Autoklaven bei einem Druck von 5 bis 8 bar unter dem Rückfluss eines Alkohols in Gegenwart einer Base, ausgewählt aus Natrium- oder Kaliumacetat, Natrium-, Kalium- oder Lithiumhydroxid, Kaliumcarbonat, Natriummethanolat oder Natriumethanolat, allein oder im Gemisch mit einer anderen dieser Basen, und dann
d) Entschützen des acetylierten Diosmins zu Diosmin durch Erhitzen in Gegenwart einer Base, ausgewählt aus Natrium-, Kalium- oder Lithiumhydroxid, Kaliumcarbonat, Natriummethanolat oder Natriumethanolat, allein oder im Gemisch mit Natrium- oder Kaliumacetat,
e) Reinigung durch Base/Säure-Behandlung.

2. Verfahren nach Anspruch 1, wobei das erhaltene Diosmin andere Flavonoide enthält.

3. Verfahren nach einem der Ansprüche 1 oder 2, wobei das erhaltene Diosmin weniger als 0,6 % 6-Iododiosmin und weniger als 3,0 % Isorhoifolin enthält.

4. Verfahren nach einem der Ansprüche 1 bis 3, wobei die Menge an Essigsäureanhydrid zwischen 8 und 10 molaren Äquivalenten, bezogen auf das eingesetzte Hesperidin, beträgt.

5. Verfahren nach einem der Ansprüche 1 bis 4, wobei die Acetylierungsreaktion (a) bei einer Temperatur zwischen 40°C und 135°C durchgeführt wird.

6. Verfahren nach einem der Ansprüche 1 bis 5, wobei der loddonor ausgewählt ist aus NaI/H₂O₂, KI/H₂O₂, TBAI/H₂O₂ und NaI/I₂ /H₂O₂.

7. Verfahren nach Anspruch 6, wobei der Joddonor Nal in einer Menge von 0,05 bis 0,2 Moläquivalenten, bezogen auf das eingesetzte Hesperidin, ist.

8. Verfahren nach einem der Ansprüche 1 bis 7, wobei die Menge an Wasserstoffperoxid 1,0 bis 1,2 Moläquivalente, bezogen auf das eingesetzte Hesperidin, beträgt.

9. Verfahren nach einem der Ansprüche 1 bis 8, wobei das nach dem Oxidationsschritt (b) erhaltene acetylierte Diosmin durch Ausfällung in Wasser isoliert wird, bevor es in Schritt c) eingesetzt wird.

10. Verfahren nach einem der Ansprüche 1 bis 9, wobei die für Schritt c) verwendete Base Natrium- oder Kaliumhydroxid in wässriger Lösung, Natrium- oder Kaliumacetat in wässriger Lösung oder ein Gemisch aus Natrium- oder Kaliumhydroxid und Natrium- oder Kaliumacetat in wässriger Lösung ist.

11. Verfahren nach einem der Ansprüche 1 bis 10, wobei der für Schritt c) verwendete Alkohol Methanol, Ethanol oder Isopropanol ist.

12. Verfahren nach einem der Ansprüche 1 bis 11, wobei die in Schritt (c) verwendete Menge an Base zwischen 0,5 und 2,5 Moläquivalenten, bezogen auf das eingesetzte Hesperidin, beträgt.

13. Verfahren nach einem der Ansprüche 1 bis 12, wobei die Menge der in Deacetylierungsschritt (d) zugesetzten Base zwischen 2 und 4,5 Moläquivalenten, bezogen auf das eingesetzte Hesperidin, beträgt.

## Claims

1. Method for the preparation of diosmin by:
a) acetylation of hesperidin,
b) oxidation of acetylated hesperidin into acetylated diosmin by an iodine donor at a temperature of 90 to 120°C,
c) heating of the acetylated diosmin, in an autoclave at a pressure of 5 to 8 bar, under reflux of an alcohol, in the presence of a base chosen from sodium or potassium acetate, sodium, potassium or lithium hydroxide, potassium carbonate, sodium methanolate or sodium ethanolate, alone or in a mixture with another of these bases, then
d) deprotection of the acetylated diosmin into diosmin by heating in the presence of a base chosen from sodium, potassium or lithium hydroxide, potassium carbonate, sodium methanolate or sodium ethanolate, alone or in a mixture with sodium or potassium acetate,
e) purification by base/acid treatment.

2. A method according to claim 1, wherein the diosmin obtained contains other flavonoids.

3. The method according to any one of claims 1 or 2, wherein the diosmin obtained contains less than 0.6% 6-iododiosmin and less than 3.0% isorhoifolin.

4. The method according to any one of claims 1 to 3, wherein the amount of acetic anhydride is between 8 and 10 molar equivalents relative to the hesperidin employed.

5. The method according to any one of claims 1 to 4, wherein the acetylation reaction (a) is carried out at a temperature between 40°C and 135°C.

6. The method according to any one of claims 1 to 5, wherein the iodine donor is selected from NaI/H₂O₂, KI/H₂O₂, TBAI/H₂O₂ and NaI/I₂ /H₂O₂.

7. The method according to claim 6, wherein the iodine donor is Na1 in an amount of from 0.05 to 0.2 molar equivalents relative to the hesperidin employed.

8. The method according to any one of claims 1 to 7, wherein the amount of hydrogen peroxide is from 1.0 to 1.2 molar equivalents relative to the hesperidin employed.

9. The method according to any one of claims 1 to 8, wherein the acetylated diosmin obtained at the end of oxidation step (b) is isolated by precipitation in water before being employed in step c) .

10. The method according to any one of claims 1 to 9, wherein the base used for step c) is sodium or potassium hydroxide in aqueous solution, sodium or potassium acetate in aqueous solution, or a mixture of sodium or potassium hydroxide and sodium or potassium acetate in aqueous solution.

11. The method according to any one of claims 1 to 10, wherein the alcohol used for step c) is methanol, ethanol or isopropanol.

12. The method according to any one of claims 1 to 11, wherein the amount of base used in step (c) is between 0.5 and 2.5 molar equivalents relative to the hesperidin employed.

13. The method according to any one of claims 1 to 12, wherein the amount of base added in the deacetylation step (d) is between 2 and 4.5 molar equivalents relative to the hesperidin employed.
